# EUROPEAN PATENT SPECIFICATION

(11) **EP 1 745 768 B1**
(45) Date of publication and mention of the grant of the patent: **08.09.2010**
(21) Application number: 06014939.0
(22) Date of filing: 18.07.2006
(51) Int. Cl.: A61H 33/06

(54) **Warm bath apparatus**
Gerät für warme Bäder
Appareil pour bains tièdes

(30) Priority: 22.07.2005 JP 2005213242
(43) Date of publication of application: 24.01.2007
(73) Proprietor: Panasonic Electric Works Co., Ltd., Kadoma-shi Osaka (JP)
(72) Inventor: Ujiie, Yoshihiko, Kadoma-shi Osaka 571-8686 (JP); Osada, Koji, Kadoma-shi Osaka 571-8686 (JP)
(74) Representative: Appelt, Christian W.

(56) References cited:
- DE-A1- 3 400 836
- GB-A- 2 056 852
- JP-A- 2003 310 699
- US-A- 3 772 713
- US-A- 6 157 005

## Description

### BACKGROUND OF THE INVENTION

### 1. Field of the Invention

The present invention relates to a warm bath apparatus that can warm a portion of a body to promote blood circulation and to improve coldness, and can further apply hot and cold stimulation to promote blood circulation.

### 2. Description of the Related Art

While sauna therapy has conventionally been chosen as a method to promote blood circulation and to improve coldness, a method for partially and easily warming a portion of a body has been demanded in recent years.

As an apparatus for partially warming a portion of a body, apparatuses that warm a hand or a foot bathed in hot water to promote blood circulation, such as a foot bath or a hand and foot bath, have been proposed. As a bathing method known as alternate hot and cold bathing, it is known to further promote blood circulation according to an application of hot stimulation and cold stimulation alternately. A technique disclosed in Japanese Patent Application Laid-open (JP-A) No. H9-224863 is known as an application of such a bathing method. In a foot bath apparatus described in JP-A No. H9-224863, hot bathing and cold bathing are made possible by using both a hot bathtub and a cold bathtub. In the conventional foot bath apparatus, however, since cold bathing and hot bathing are performed using cold water and hot water respectively, there is a problem that the apparatus becomes large. Furthermore, since it is necessary to prepare a large amount of hot water and cold water and to drain them after use, it is inconvenient. Even if a feed pipe and a drain pipe are provided for saving troubles at the time of supplying and draining water, there are still problems such that an apparatus itself becomes large and a place for using the apparatus is limited due to piping.

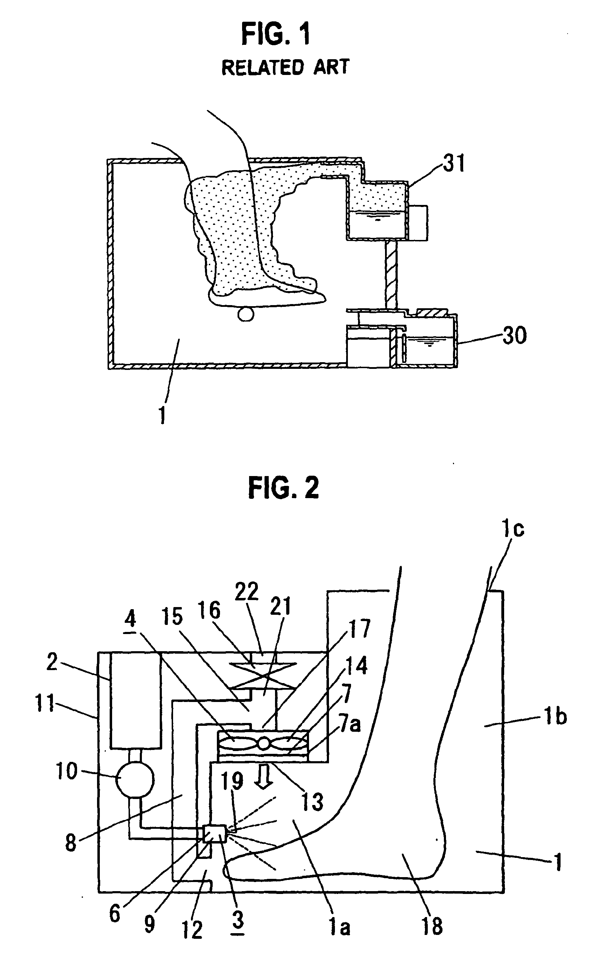

A hot bath apparatus for achieving an effect of hyperthermia is known from JP-A No. 2003-310699. According to this application, it is proposed to provide a hot mist generation tank 30 for generating hot mist and a cold mist generation tank 31 for generating cold mist in a main unit 11 having a bathtub 1, to perform alternate hot and cold bathing by a hot bath utilizing hot mist and by a cold bath utilizing cold mist alternately, as shown in Fig. 1. In this conventional example, however, since the hot mist generation tank 30 and the cold mist generation tank 31 have to be provided for the alternate hot and cold bathing, there are problems such that the apparatus itself is large-sized and is complicated in configuration. Even though the hot mist and the cold mist are supplied alternately to allow alternate hot and cold bathing, this configuration alone does not make the difference between hot and cold stimulations distinct. Therefore, a sufficient blood circulation effect cannot be obtained. Accordingly, there is a demand for further enhancement of the blood circulation effect.

From GB 2 056 852 A a warm bath apparatus in the form of a facial sauna device is known, which comprises a bathtub in which a portion of a body can be put; a water storage tank; a hot mist producing unit that changes water in the water storage tank to hot mist to apply hot stimulation by hot mist to the portion of a body put into the bathtub; and an external air introducing unit with an external air introducing path, a fan, a heater.

It is an object of the present invention to provide a further enhanced warm bath apparatus that has a simple configuration and is convenient to use, and can realize high-blood circulation promoting effect by hot and cold stimulations.

### SUMMARY OF THE INVENTION

The above-mentioned problem is solved by a warm bath apparatus according to claim 1, claims 2 to 8 refer to specifically advantageous realizations of the warm bath apparatus as defined in claim 1.

In order to solve the above problems, a warm bath apparatus according to the present invention comprises a bathtub 1 into which a portion of a body can be put, a water storage tank 2, a hot mist producing unit 3 that changes water in the water storage tank 2 to hot mist to apply hot stimulation thereby to the portion of a body put into the bathtub 1, and an external air introducing unit 4 that supplies external air in the bathtub 1 through air supply or air exhaust to apply cold stimulation to the portion of a body put into the bathtub 1.

With this configuration, hot mist is produced by the hot mist producing unit 3 so that blood circulation can be promoted by applying hot mist to the portion of a body to stimulate the same. During cold bathing, air in the bathtub 1 is discharged and external air is simultaneously introduced in the bathtub 1 by the external air introducing unit 4, so that the portion of a body covered with hot mist by hot bathing is cooled and dried by external air which is not as hot as the air in the bathtub 1 and has moisture lower than that of the air in the bathtub 1, and vaporization heat is drawn from the portion of a body when water covering the portion of a body is vaporized. As a result, cold stimulation can be applied to the portion of a body. Accordingly, since alternate hot and cold stimulation is applied to the portion of the body, the blood circulation promoting effect is further improved. In addition, since the external air introducing unit 4 that supplies external air in the bathtub 1 through air supply and air exhaust to apply cold stimulation to a portion of a body put into the bathtub 1 in order to apply cold stimulation, it is unnecessary to provide a plurality of tanks that are required in the conventional techniques, so that hot and cold bathing can be conducted conveniently at any place with a simple configuration and with easier preparation and cleanup.

It is preferable that the hot mist producing unit 3 is a steam generator 5 for heating water to vaporize the same.

With such a configuration, steam is produced by the steam generator 5 so that hot bathing can be conducted, and environmental control in the bathtub 1 can be conducted by only controlling the steam generator 5 such as a boiler, so that a complicated control unit is not required.

It is preferable that the hot mist producing unit 3 includes a cold mist producing unit 6, and a heating unit 7 for heating cold mist produced by the cold mist producing unit 6.

With such a configuration, a portion of a body put into the bathtub 1 can be warmed by producing cold mist by the cold mist producing unit 6 and then heating the cold mist in the bathtub 1 by the heating unit 7 to produce hot mist, or the portion of a body put into the bathtub 1 can be warmed by producing cold mist by the cold mist producing unit 6 and then heating the same by the heating unit 7 to produce hot mist and supply the same to the bathtub 1. As a result, a possibility such that high-temperature water is supplied to the bathtub 1 due to any trouble is avoided, and an apparatus which is safe and is manufactured at low cost can be obtained.

It is preferable that the heating unit 7 is provided in a circulation path 8 for circulating air inside the bathtub 1.

With this configuration, warming is performed by circulating air in the bathtub 1. Accordingly, when cold mist is produced, hot air allows hot bathing so that a whole portion of a body put into the bathtub 1 can be subjected to hot stimulation, and when hot mist is produced, hot air is applied additionally during hot bathing so that the portion of a body put into the bathtub 1 can be further warmed. Therefore, this configuration makes the difference between hot and cold distinct, and a higher blood circulation promoting effect can be obtained.

It is preferable that the cold mist producing unit 6 includes a pump 10 and a nozzle 9.

With such a configuration, water in the water storage tank 2 can be sucked by the pump 10 so as to be supplied in the bathtub 1 via the nozzle 9 as mist. Accordingly, the mist can be produced using an apparatus with a simple configuration and at low cost.

It is preferable that the cold mist producing unit 6 includes an ultrasonic device.

Since mist is produced by ultrasonic wave in this manner, particle diameters of mist particles can be reduced. Accordingly, mist is spread evenly over a whole portion of a body put into the bathtub 1, which results in an advantage such as promotion of the hot bathing effect.

It is preferable that a controller which controls to jet cold mist during cold stimulation is provided.

With such a configuration, a portion of a body put into bathtub 1 is further cooled during cold stimulation. Therefore, this configuration makes the difference between hot and cold distinct, and blood circulation can be promoted. When the hot mist producing unit 3 includes the cold mist producing unit 6, and the heating unit 7 for heating cold mist produced by the cold mist producing unit 6, coldmist can be supplied during cold stimulation by actuating the cold mist producing unit 6 which is one component of the hot mist producing unit 3, and it is unnecessary to provide a new or additional cold mist producing unit for cold stimulation, so that the apparatus can be simplified in structure and be made compact.

It is preferable that a heating unit 7 for heating introduced external air is provided in the external air introducing unit 4.

With such a configuration, hot air whose moisture is lower than that of air in the bathtub 1 and which is obtained by heating external air by the heating unit 7 can be supplied into the bathtub 1 in a final stage of hot bathing or hot and cold bathing of a portion of a body, so that the interior of the bathtub 1 can be dried after the hot bathing or hot and cold bathing. The portion of a body put into the bathtub 1 can be accordingly dried by the hot air and the portion can be taken out from the bathtub 1 in a warmed state. Therefore, it is unnecessary to wipe the portion, and a trouble such as wiping dew condensation water in the bathtub 1 is omitted, which results in convenience.

### BRIEF DESCRIPTION OF THE DRAWINGS

Fig. 1 is a cross-sectional view of a conventional technique;
Fig. 2 is a cross-sectional view of an embodiment of the present invention;
Fig. 3 is a cross-sectional view of the embodiment showing a flow of air during hot bathing;
Fig. 4 is a cross-sectional view of the embodiment showing a flow of air during cold bathing;
Fig. 5 is a cross-sectional view of another embodiment of the present invention; and
Fig. 6 is a cross-sectional view of still another embodiment of the present invention.

### DETAILED DESCRIPTION OF THE PREFERRED EMBODIMENTS

Embodiments of the present invention will be explained below in detail with reference to the accompanying drawings.

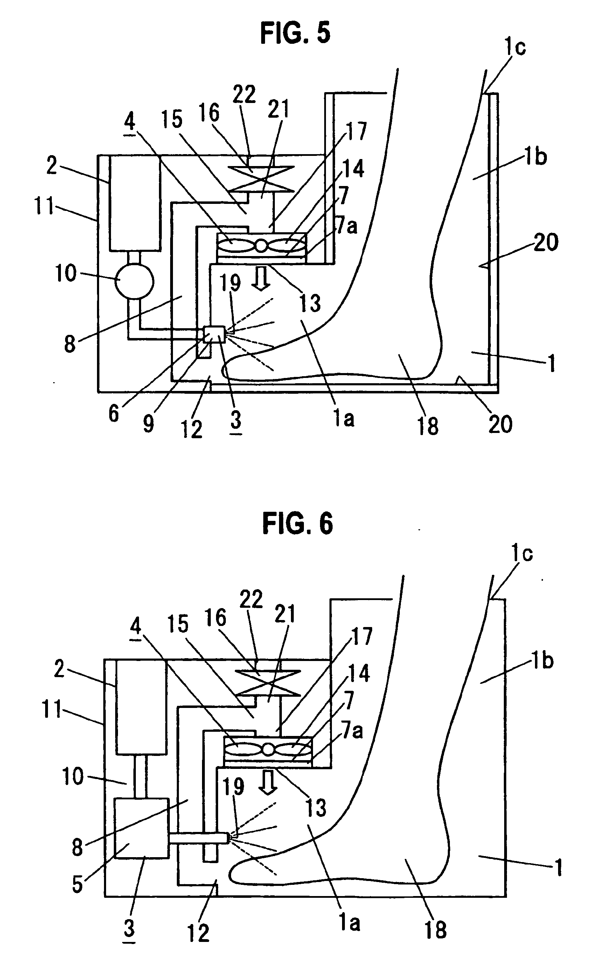

Figs. 2 to 4 show an embodiment of the present invention, Fig. 5 shows another embodiment, and Fig. 6 shows still another embodiment. While examples of a foot bath apparatus for promoting blood circulation in feet of a body as a warm bath apparatus are shown in the embodiments shown in Figs. 2 to 4, Fig. 5, and Fig. 6, a portion of a body whose blood circulation should be promoted is not limited to feet. The portion of a body can be hands, a face, or a head for promoting hair growth.

An embodiment of the present invention will be explained with reference to Figs. 2 to 4.

As shown in Fig. 2, a foot bath apparatus serving as a warm bath apparatus includes a bathtub 1 where a foot 18 that is a portion of a body is put into a main unit 11 and a circulation path 8 for circulating air inside the bathtub 1. Further, the foot bath apparatus is provided with a hot mist producing unit 3 for applying hot stimulation by hot mist to a portion (a foot) of a body put into the bathtub 1, and an external air introducing unit 4 for supplying external air into the bathtub 1 through air supply or air exhaust to apply cold stimulation to the portion of a body put into the bathtub 1.

The bathtub 1 is formed in an approximately L shape in vertical section, and it has a foot insertion portion 1a at a lower portion and an insertion cylindrical portion 1b whose width in a longitudinal direction thereof is narrower than the foot insertion portion 1a at an upper portion, an upper end opening of the insertion cylindrical portion 1b constituting an insertion port 1c for foot insertion.

In the circulation path 8, one end portion thereof that is an upstream side end constitutes a suction port 12 opened at a distal end lower portion of the foot insertion portion 1a of the bathtub 1 and the other end portion that is a downstream side end is an air blow port 13 opened at a front upper portion of the foot insertion portion 1a of the bathtub 1.

An external air introducing path 21 is provided in the main unit 11, one end thereof constituting an external air introducing port 22 opened externally and a downstream side thereof also serving as a downstream side of the circulation path 8, so that a midway through the external air introducing path 21 communicates with a midway through the circulation path 8 at a portion of a communicating portion 15 and a portion of the external air introducing path 21 positioned downstream of the communicating portion 15 constitutes a dual purpose portion 17 serving as both a downstream portion of the circulation path 8 and a downstream portion of the external air introducing path 21. Therefore, a downstream end portion of the external air introducing path 21 constitutes the air blow port 13 which is a downstream end portion of the circulation path 8. An opening and closing valve 16 is provided between the external air introducing port 22 and the communicating portion 15 of the external air introducing path 21.

A fan 14 for blowing air into the bathtub 1 and a heater 7a constituting a heating unit 7 are provided in the dual purpose portion 17 having the air blow port 13.

A water storage tank 2 is provided in the main unit 11, so that water in the water storage tank 2 is supplied to a nozzle 9 by a pump 10 to produce cold mist and supply the same in the bathtub 1. That is, the pump 10 and the nozzle 9 constitute a cold mist producing unit 6 in this embodiment.

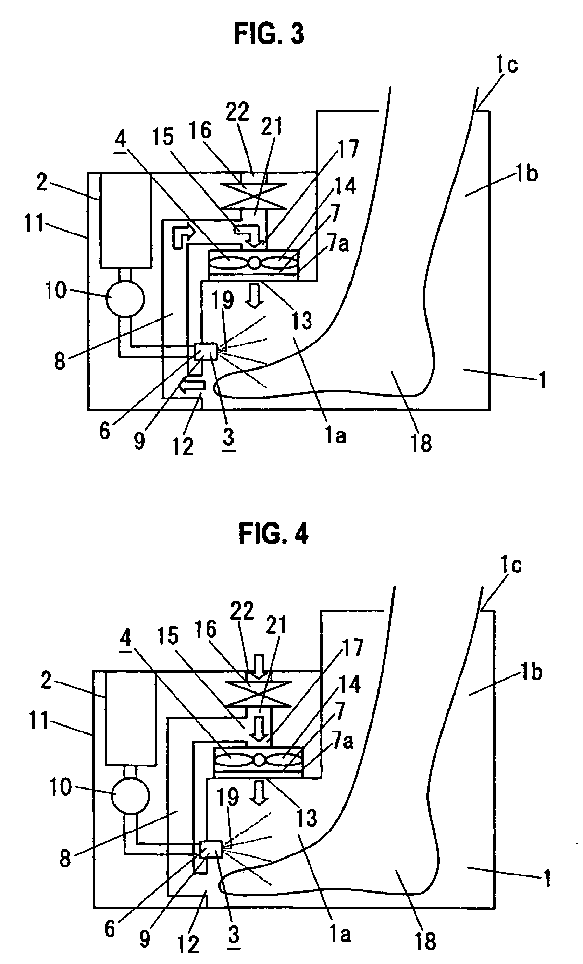

When alternate hot and cold bathing is performed in the foot bath apparatus which is the warm bath apparatus, first, a predetermined amount of water is charged in the water storage tank 2 and the water storage tank 2 is set in the main unit 11. The foot 18 is put into the bathtub 1 from the insertion port 1c, and the alternate hot and coldbathing is started in this state by starting an operation unit (not shown). The alternate hot and cold bathing is first started from hot bathing. That is, the fun 14 is actuated and the heater 7a that is the heating unit 7 is supplied with power, and the controller (not shown) controls so that the opening and closing valve 16 provided in the external air introducing path 21 is maintained in a closed state. As a result, as shown by arrow in Fig. 3, air in the bathtub 1 is sucked from the suction port 12 into the circulation path 8 to be heated by the heating unit 7, so that it is fed in the bathtub 1 via the air blow port 13 as hot air, and heat is applied to the foot 18 by the hot air. The pump 10 is then actuated, water in the water storage tank 2 is fed to the nozzle 9 by the pump 10, and water is changed to mist (cold mist) when it passes through the nozzle 9 to be jetted from a mist jetting port 19 provided at a distal end of the nozzle 9 into the bathtub 1. Mist jetted into the bathtub 1 puts on the foot 18. At that time, since air in the bathtub 1 is circulated by the fan 14, the jetted mist is agitated in the bathtub 1 to be made easier to put on the foot 18. The mist put on the foot is heated by hot air blown in the bathtub 1 to change to hot mist and the foot 18 is applied with heat via the hot mist. Therefore, the foot 18 becomes warmer as compared with the case of utilizing only hot air, so that effective hot bathing can be achieved by heated hot mist and hot air.

In the embodiment, the cold mist producing unit 6 including the pump 10 and the nozzle 9 and the hot air generating unit including the fan 14 and the heater 7a that is the heating unit 7 constitute the hot mist producing unit 3 that changes water in the water storage tank 2 to hot mist.

While, in the embodiment shown in Fig. 2, the nozzle 9 is provided at a position where it directly jets mist in the bathtub 1, the position of the nozzle 9 is not limited to the above example. For example, by positioning the nozzle 9 upstream of the fan 14 in the circulation path 8 (at the dual purpose portion 17 or upstream thereof), the mist jetted from the nozzle 9 can be heated more easily by the heater 7a that is the heating unit 7 when the fan 14 sends air.

While the heater 7a provided at the dual purpose portion 17 constitutes the heating unit 7 in the embodiment, the heating unit 7 is not limited to this configuration. For example, the heater 7a serving as a heating unit 7 is embedded in a wall portion of the bathtub 1, so that air in the bathtub 1 is heated by the heater 7a so as to heat the cold mist jetted from the nozzle 9 to be hot mist.

In the present embodiment, the hot mist producing unit 3 is constituted such that cold mist produced by the cold mist producing unit 6 is heated by the fan 14 and the hot air generating unit including the heater 7a which is the heating unit 7 in order to produce hot mist, however, the hot mist producing unit is not limited to this configuration. For example, the heater 7a which is the heating unit 7 is provided inside the water storage tank 2, water in the tank 2 is heated to a proper temperature by the heater 7a, and then feeding hot water to the nozzle 9 by the pump 10 to generate hot mist and supply the same in the bathtub 1.

Control is made such that, after such hot bathing as described above is performed for a predetermined time, switching to cold bathing is performed automatically. At a time of the cold bathing, the heater 7a that is the heating unit 7 turns OFF and simultaneously the opening and closing valve 16 provided in the external air introducing path 21 switches from open to close. External air enters from the external air introducing port 22 as shown by arrow in Fig. 4, where the air is blown from the air blow port 13 into the bathtub 1 by the fan 14. Since the external air introduced into the bathtub 1 in this manner has a temperature and a moisture lower than those of air in the bathtub 1, the external air cools and dries the foot 18 covered with hot mist and water covering the foot 18 draws vaporization heat from the foot when it is vaporized so that the foot is cold-stimulated. In this case, by further producing cold mist utilizing the cold mist producing unit 6 to jet the same into the bathtub 1, the foot is further cooled. Accordingly, a difference in temperature between hot and cold can be widened in the alternate hot and cold stimulation, allowing the difference therebetween become more distinct.

In the above example, a foot is cooled by external air introduced through the external air introducing unit 4 constituted such that external air sucked from the air blow port 13 is supplied in the bathtub 1 by the fan 14. That is, by supplying the external air into the bathtub 1 utilizing the fan 14 that is the air supplying unit during cold bathing, the external air is introduced into the bathtub 1 so that wind of the external air is blown to the foot 18 to cool the foot 18. Alternatively, by discharging air in the bathtub 1 utilizing an exhaust fan provided in the bathtub 1 as an exhaust unit, external air is sucked in the bathtub 1 so that the external air can be blown on the foot for cooling the same.

Control is made such that, after cold bathing for a predetermined time is performed in the above manner, switching to hot bath is performed automatically. By repeating the hot bathing and the cold bathing alternately, the blood circulation promoting effect can be improved. Peripheral blood vessels dilate during hot bathing, while they constrict during cold bathing, so that the peripheral blood vessels are trained by repeating the dilation and constriction alternately according to the alternate hot and cold stimulation. Therefore, even in a person whose blood vessels less likely dilate so that he/she is poor in blood circulation, his/her blood vessels dilate easily, which results in improvement in blood circulation. By repeatedly using the foot bath apparatus on consecutive days, coldness and various symptoms due to poor blood circulation are improved by blood circulation promotion based on the alternate hot and cold bathing.

After termination of the alternate hot and cold bathing, by activating the fan 14 in an opened state of the opening and closing valve 16 provided in the external air introducing path 21 and supplying power to the heater 7a that is the heating unit 7, external air is heated by the heating unit 7 to be supplied in the bathtub 1, so that the foot 18 put in a wet state in the bathtub 1, and an inner wall and a bottom wall of the bathtub 1 are dried. Unless the drying function is provided in the foot bath apparatus, the hot foot 18 remains wet when it is drawn out of the bathtub 1, and cool feeling remains, which results in deterioration of sense of use. Since dew condensation water puts on the inner wall and the bottom portion in the bathtub 1, if it is left as it is, sanitary deterioration may occur, so that such maintenance work as wiping of dew condensation water is required.

In the embodiment, since the cold mist producing unit 6 includes the pump 10 and the nozzle 9, the configuration thereof is much simplified, and an amount of mist can be controlled according to flow rate control of the pump 10, time control on an operating time or a stopping time, or a diameter of the nozzle 9, which results in provision of a low cost apparatus.

The cold mist producing unit 6 can be constituted to have an ultrasonic wave device (not shown). When a system utilizing ultrasonic wave is adopted in the cold mist producing unit 6 in this manner, the particle diameter of mist can be reduced, so that the mist spreads in the whole space in the bathtub 1 evenly and the mist puts on the foot 18 evenly. A sense of warmth is therefore increased during hot bathing, while the whole foot 18 becomes cold evenly during cold bathing. At this time, it does not particularly mean that the smaller the particle diameter of mist, the better the foot bath apparatus is. Since excessively small mist particles do not put on a skin surface of the foot and sense of actual use lowers, mist having a proper particle diameter is desirable. Means for reducing the particle diameter of mist particles is not limited to the system utilizing an ultrasonic wave, but it can be a discharging system or the like.

Another embodiment of the present invention will be explained with reference to Fig. 5.

While a foot bath apparatus of the embodiment is similar to that of the embodiment shown in Fig. 2 in a basic configuration, the former is different from the latter in a characteristic such that a heater 20 radiating far-infrared rays is provided in the bathtub 1 of the foot bath apparatus shown in Fig. 2, as shown in Fig. 5. In Fig. 5, a carbon film heater used as the heater 20 is put on almost all area of an inner face of the bathtub 1 to radiate far-infrared rays in the bathtub 1, but the present invention is not limited to this configuration. Far-infrared rays can be radiated in the bathtub 1 by attaching the heater 20 on an outer surface of the bathtub 1 or embedding the heater 20 in a wall of the bathtub 1 and applying far-infrared ray paint on an inner face of the bathtub 1.

The operation in the embodiment, when alternate hot and cold bathing is performed in the foot bath apparatus, is similar to that of the embodiment shown in Figs. 2 to 4. However, the former is different from the latter in that heat can be applied to the foot 18 during hot bathing not only by heat transfer or convective flow from mist and hot air via water content and air but also by radiation of far-infrared rays due to power supplying to the heater 20 radiating far-infrared rays so that the hot bathing effect can be further improved. In the embodiment, therefore, a difference in temperature between hot and cold can be widened in alternate hot and cold stimulation so that the difference between hot and cold can be made distinct. At this time, since the heat received by the foot 18 changes (a sense of warmth changes) depending on a surface temperature of the heater 20 and a distance from the heater 20 to the foot 18, it is necessary to set a surface temperature (wattage) of the heater 20 to radiate far-infrared rays at a level where burn is not caused. Since low temperature burn may be caused due to contacting with the heater 20, there is a need to adopt a configuration that the surface of the heater 20 does not contact with the foot 18 directly.

When alternate hot and cold bathing is performed, external air is introduced into the bathtub 1 to lower a temperature inside the bathtub 1 temporarily during cold bathing. Therefore, if power of the heat is insufficient at a starting time of hot bathing subsequent to the cold bathing, much time is required to raise a temperature inside the bathtub 1, and the bathtub 1 may be hard to be warmed. However, by supplying power to the heater 20 radiating far-infrared rays in addition to hot mist, a time period required until a user feels the heat can be reduced.

Still another embodiment of the present invention will be explained next with reference to Fig. 6.

While the hot mist producing unit 3 includes the cold mist producing unit 6 and the heating unit 7 for heating cold mist produced by the cold mist producing unit 6 in the embodiment shown in Fig. 2, the hot mist producing unit 3 includes a steam generating unit 5 which heats water to vaporize the same in this embodiment, which is different from the embodiment shown in Fig. 2. Other basic configurations of the embodiment are similar to that of the embodiment shown in Fig. 2.

That is, in the embodiment, a boiler constituting the steam generating unit 5 is provided in the main unit 11, so that water in the water storage tank 2 is supplied to the boiler, steam (hot mist) is produced by the boiler, and hot mist produced is supplied into the bathtub 1 from a mist jetting port 19.

The operation in the embodiment, when alternate hot and cold bathing is performed in the foot bath apparatus, is similar to that of the embodiment shown in Figs. 2 to 4, but the former is different from the latter that, after the water storage tank 2 is charged with a predetermined amount of water and it is set in the main unit 11, a predetermined amount of water is flowed from the water storage tank 2 in the boiler constituting the steam generating unit 5, water in the boiler is heated and vaporized by supplying power to the boiler simultaneously with the start of hot bathing, and the vaporized water is flowed from the mist jetting port 19 into the bathtub 1 as steam (hot mist). As a result, hot mist flowed into the bathtub 1 puts on the foot 18 to warm it. At this time, environment such as a temperature inside the bathtub 1 is almost determined according to boiler controlling.

By supplying power to the fan 14 and the heater 7a, hot air is fed into the bathtub 1 via the air blow port 13, so that effective hot bathing based on the steam (hot mist) and hot air can be achieved. Accordingly, a difference in temperature between hot and cold can be widened in the alternate hot and cold stimulation, which makes the difference between hot and cold distinct. In the present embodiment, the heater 20 radiating far-infrared rays can be provided like the embodiment shown in Fig. 5, where hot bathing up to a relatively high temperature range is made possible.

In each of the embodiments described above, the present invention is not limited to alternate hot and cold bathing. The invention can be constituted that a user can select using the alternate hot and cold bathing or only hot bathing. In this case, by setting a mode for alternate hot and cold bathing and a mode for only hot bathing, the user can select one of the two modes. When the mode for alternate hot and cold bathing is selected, the operation for alternate hot and cold bathing is performed as described above. When an operation for only hot bathing is selected, only an operation for hot bathing (the same operation as the one for hot bathing in the alternate hot and cold bathing operation) is performed to warm the foot 18, so that blood circulation is promoted by only hot bathing.

According to the present invention, alternate hot and cold stimulation of hot stimulation provided by hot mist and cold stimulation, which is provided by cooling and drying a portion of a body covered with hot mist by introduction of external air so that vaporization heat is drawn from the portion of a body when water covering the portion of a body is vaporized, can be applied to the portion of a body. As a result, a high blood circulation promoting effect can be achieved. Furthermore, since the external air introducing unit, which supplies external air into the bathtub through air supply and air exhaust to apply cold stimulation to the portion of a body put into the bathtub in order to apply cold stimulation to the portion of a body, is provided, it is unnecessary to provide a plurality of tanks that are required in the conventional techniques, so that hot and cold bathing can be conducted conveniently at any place with a simple configuration and with easier preparation and cleanup.

While the embodiment of the present invention has been described above, the invention is not limited to the above embodiment and changes and modifications can be made within the scope of the present invention as defined in the appended claims.

## Claims

1. A warm bath apparatus comprising;
a bathtub (1) in which a portion of a body can be put;
a water storage tank (2);
a hot mist producing unit (3) that changes water in the water storage tank (2) to hot mist to apply hot stimulation by hot mist to the portion of a body put into the bathtub (1); and
an external air introducing unit (4) to apply cold stimulation to the portion of a body put into the bathtub, with an external air introducing path (21), a fan (14), a heater (7) and an opening and closing valve (16) provided in said external air introducing path (21);
a circulation path (8) for circulating air; and
a dual purpose portion (17) that serves as both a downstream portion of said external air introducing path (21) and a downstream portion of said circulation path (8);
wherein the fan (14) and the heater (7) are disposed inside said dual purpose portion (17).

2. The warm bath apparatus according to claim 1, wherein the hot mist producing unit (3) is a steam generating unit (5) that heats water to vaporize the same.

3. The warm bath apparatus according to claim 1, wherein the hot mist producing unit (3) includes a cold mist producing unit (6) and a heating unit (7) that heats cold mist produced by the cold mist producing unit (6).

4. The warm bath apparatus according to claim 3, wherein the cold mist producing unit (6) includes a pump (10) and a nozzle (9).

5. The warm bath apparatus according to claim 3, wherein the cold mist producing unit (6) includes an ultrasonic device.

6. The warm bath apparatus according to any of the claims 3 to 5, further comprising a controller that controls to jet cold mist during cold stimulation.

7. The warm bath apparatus according to claim 1, wherein, after application of hot stimulation to the portion of a body conducted by the hot mist producing unit (3) and application of cold stimulation to the portion of a body conducted by the external air introducing unit (4) are terminated, external air is heated by the heating unit (7) and is supplied into the bathtub (1) by the fan (14).

8. The warm bath apparatus according to claim 1, comprising a heater (20) attached on an inner face of the bathtub (1) to radiate far-infrared rays in the bathtub (1).

## Patentansprüche

1. Warmbadgerät, umfassend:
eine Badewanne (1), in die ein Teil eines Körpers gelegt werden kann;
einen Wasserspeicherbehälter (2);
eine Heißnebelerzeugungseinheit (3), die das Wasser im Wasserspeicherbehälter (2) in heißen Nebel umwandelt, um einen Hitzereiz durch den Heißnebel auf den Teil eines Körpers anzuwenden, der in die Badewanne (1) gelegt ist; und
eine Außenluftzuführungseinheit (4), um einen Kältereiz auf den Teil eines Körpers anzuwenden, der in die Badewanne gelegt ist, mit einer Außenluftzuführungsleitung (21), einem Gebläse (14), einem Heizer (7) sowie einem Öffnungs- und Schließventil (16), das in der Außenluftzuführungsleitung (21) vorgesehen ist;
eine Zirkulationsleitung (8) zum Umwälzen der Luft;
und einen Bereich (17), der zwei Zwecken dient und der sowohl als ein Downstream-Bereich der Außenluftzuführungsleitung (21) als auch als ein Downstream-Bereich der Zirkulationsleitung (8) dient;
wobei das Gebläse (14) und der Heizer (7) innerhalb des Bereichs (17), der zwei Zwecken dienen kann, angeordnet sind.

2. Warmbadgerät nach Anspruch 1, wobei die Heißnebelerzeugungseinheit (3) eine Dampferzeugungseinheit (5) ist, die Wasser erhitzt, um es zu verdampfen.

3. Warmbadgerät nach Anspruch 1, wobei die Heißnebelerzeugungseinheit (3) eine Kaltnebelerzeugungseinheit (6) und eine Heizeinheit (7) umfasst, die den Kaltnebel erhitzt, der durch die Kaltnebelerzeugungseinheit (6) erzeugt wird.

4. Warmbadgerät nach Anspruch 3, wobei die Kaltnebelerzeugungseinheit (6) eine Pumpe (10) und eine Düse (9) umfasst.

5. Warmbadgerät nach Anspruch 3, wobei die Kaltnebelerzeugungseinheit (6) ein Ultraschallbauteil umfasst.

6. Warmbadgerät nach einem der Ansprüche 3 bis 5, das ferner ein Steuergerät umfasst, das den Ausstoß von Kaltnebel während der Kältereizeinwirkung steuert.

7. Warmbadgerät nach Anspruch 1, wobei dann, wenn die Anwendung des Hitzereizes auf den Teil eines Körpers, die durch die Heißnebelerzeugungseinheit (3) ausgeführt wird, und die Anwendung des Kältereizes auf den Teil eines Körpers, die durch die Außenluftzuführungseinheit (4) ausgeführt wird, abgeschlossen sind, die Außenluft durch die Heizeinheit (7) erwärmt und durch das Gebläse (14) in die Badewanne (1) geblasen wird.

8. Warmbadgerät nach Anspruch 1, einen Heizer (20) umfassend, der an einer Innenfläche der Badewanne (1) angebracht ist, um langwellige Infrarotstrahlen in die Badewanne (1) abzustrahlen.

## Revendications

1. Appareil pour bains tièdes comprenant;
une baignoire (1) dans laquelle on peut placer une partie d'un corps;
un réservoir (2) de stockage d'eau;
une unité (3) de production de brume chaude qui change l'eau dans le réservoir (2) de stockage d'eau en une brume chaude pour appliquer une stimulation chaude par la brume chaude à la partie du corps placée dans la baignoire (1); et
une unité (4) d'introduction d'air externe avec un chemin (21) d'introduction d'air externe, un ventilateur (14), un réchauffeur (7) et une vanne (16) d'ouverture et de fermeture pourvue dans ledit chemin (21) d'introduction d'air externe;
un chemin de circulation (8) pour faire circuler de l'air; et
une partie (17) à double usage qui fait office de partie en aval dudit chemin (21) d'introduction d'air externe et de partie en aval dudit chemin de circulation (8);
où le ventilateur (14) et le réchauffeur (7) sont disposés dans ladite partie à double usage (17).

2. Appareil pour bains tièdes selon la revendication 1, dans lequel l'unité (3) de production de brume chaude est une unité (5) de génération de vapeur qui chauffe l'eau pour la vaporiser.

3. Appareil pour bains tièdes selon la revendication 1, dans lequel l'unité (3) de production de brume chaude comporte une unité (6) de production de brume froide et une unité de chauffage (7) qui chauffe la brume froide produite par l'unité (6) de production de brume froide.

4. Appareil pour bains tièdes selon la revendication 3, dans lequel l'unité (6) de production de brume froide comporte une pompe (10) et une buse (9).

5. Appareil pour bains tièdes selon la revendication 3, dans lequel l'unité (6) de production de brume froide comporte un dispositif ultrasonore.

6. Appareil pour bains tièdes selon l'une quelconque des revendications 3 à 5, comprenant en outre une unité de commande qui exécute une commande afin de gicler de la brume froide durant la stimulation froide.

7. Appareil pour bains tièdes selon la revendication 1, dans lequel, après l'achèvement de l'application de la stimulation chaude à la partie d'un corps effectuée par l'unité (3) de production de brume chaude et de l'application de la stimulation froide à la partie d'un corps effectuée par l'unité (4) d'introduction d'air externe, de l'air externe est chauffé par l'unité de chauffage (7) et est alimenté dans la baignoire (1) par le ventilateur (14).

8. Appareil pour bains tièdes selon la revendication 1, comprenant un dispositif de chauffage (20) fixé sur une face interne de la baignoire (1) pour émettre des rayons infrarouges lointains dans la baignoire (1).
